# EUROPÄISCHE PATENTSCHRIFT

(11) **EP 3 065 539 B1**
(45) Veröffentlichungstag und Bekanntmachung des Hinweises auf die Patenterteilung: **16.10.2019**
(21) Anmeldenummer: 14815220.0
(22) Anmeldetag: 05.11.2014
(51) Int. Cl.: C02F 1/00, A01G 33/00, C02F 3/32, C12M 1/00, C12M 1/12, C12M 1/26

(54) **VERFAHREN ZUR ABWASSERREINIGUNG MITTELS SICH ABSETZENDER ALGENMISCHKULTUREN UND VORRICHTUNG ZU DESSEN DURCHFÜHRUNG**
METHOD FOR WASTEWATER TREATMENT BY MEANS OF MIXED ALGAE CULTURES THAT SEDIMENT AND DEVICE FOR PERFORMING SAID METHOD
PROCÉDÉ POUR L'ÉPURATION D'EAUX USÉES AU MOYEN DE CULTURES MIXTES D'ALGUES QUI SE DÉPOSENT ET DISPOSITIF POUR LA MISE EN UVRE DE CE PROCÉDÉ

(30) Priorität: 07.11.2013 DE 102013112269
(43) Veröffentlichungstag der Anmeldung: 14.09.2016
(73) Patentinhaber: Holm, Niels, 32479 Hille (DE)
(72) Erfinder: Holm, Niels, 32479 Hille (DE)
(74) Vertreter: Lobemeier, Martin Landolf
(86) Internationale Anmeldenummer: PCT/DE2014/100392
(87) Internationale Veröffentlichungsnummer: WO 2015/067243

(56) Entgegenhaltungen:
- EP-A1- 2 486 790
- WO-A2-98/45405
- DE-A1- 19 752 542
- GB-A- 2 335 199
- US-A1- 2002 034 817
- US-A1- 2010 264 094
- US-A1- 2012 021 498

## Beschreibung

Die Erfindung betrifft ein Verfahren zum Reinigen eines Abwassers mittels photosynthetisch aktiver Algen und eine Vorrichtung zur Durchführung des Verfahrens. Insbesondere betrifft die Erfindung ein Verfahren und eine Vorrichtung, die eine Abwasserreinigung mit weitgehender Nährstoffrückgewinnung unter Biomasseproduktion ermöglichen.

Die Verwendung von Algenmassen bzw. Algenmischkulturen bei unterschiedlicher Anlagenkonfigurationen zur Biomasseproduktion und/oder Abwasserreinigung, auch in Kombination mit Biogasanlagen, ist bereits bekannt; siehe beispielsweise DE 196 11 885 C1 und DE 197 21 243 C2. Die mithilfe dieser Verfahren produzierte Biomasse kann zu Nahrungsmitteln aufbereitet werden. Darüber hinaus können aber auch unterschiedliche Produkte wie Feinchemikalien, Vitamine, Farbstoffe etc. gewonnen werden.

Im Rahmen der Abwasserreinigung wird die anfallende Biomasse üblicherweise als zu entsorgender Reststoff behandelt, der beispielsweise über eine Faulung entsorgt oder als nachwachsender Rohstoff zur Biogasproduktion in Biogasanlagen eingesetzt werden kann.

Unabhängig vom Verwendungszweck muss die Algenbiomasse bei den bisherigen Kulturtechnologien immer aus den wässrigen Kulturmedien aufkonzentriert entnommen werden.

Aus verschiedenen Gründen sind nahezu alle bisherigen Groß-Kulturtechniken darauf ausgerichtet, freischwebende Algen zu kultivieren; siehe z.B. DE 197 52 542 A1. Algen sind dann als freischwebend zu bezeichnen, wenn diese weder an der Oberfläche aufschwimmen, noch sich unter Ruhebedingungen absetzen. Sie müssen daher recht aufwändig aus der wässrigen Lösung mittels Filtration oder Zentrifugation aufkonzentriert und geerntet werden.

Abgesehen von einer Reihe weiterer bei der Kultivierung von freischwebenden Algen zu berücksichtigender technologischer Probleme - wie z.B. ausreichende CO₂-Zufuhr bzw. Vermeidung einer zu starken O₂-Übersättigung, sowie dem Umgang mit stark schwankenden Lichtintensitäten und Temperaturen im Tages- und Jahresverlauf, insbesondere bei offenen Systemen - sind die immer noch viel zu teuren Erntetechniken die Hauptursache für derzeit nur sehr spärlichen großtechnischen Einsatz dieser Technologie.

Zwar ist aus der EP 1 972 602 B1 bereits ein Verfahren bekannt, mit dem zielgerichtet sowohl frei schwebende als auch sich schnell absetzende Algenpopulationen gezüchtet werden können.

Einerseits weist dieses Verfahren aber den Nachteil auf, dass das Verfahren aufgrund der offenen Betriebsweise unter den gegebenen klimatischen Bedingungen in Mittel- und Nordeuropa, wo im Winter die Außentemperatur unter den Gefrierpunkt fallen kann und/oder im Winter die Tageslichtintensitäten so gering sind, dass keine oder kaum eine Netto-Photosynthese-Produktion möglich ist, nicht ganzjährig betrieben werden kann, insbesondere auch weil die zu treffenden Gegenmaßnahmen, wie etwa künstliche Beleuchtung und/oder Aufheizung, unwirtschaftlich sind.

Andererseits zeigte ein von der DBU gefördertes Forschungsvorhaben (Az: 25907), dass eine selektive Züchtung der leicht zu erntenden, sich absetzenden Algen basierend auf dem Patent EP 1 972 602 B1 nur bei sehr kurzen hydraulischen Aufenthaltszeiten der Wasserphase möglich ist, im Übrigen aber auch bei längerer hydraulischer Aufenthaltszeit schnell zur Bildung von frei schwebende planktischen Mikroalgen führt.

Aufgabe der Erfindung ist es daher, ein Verfahren zum Reinigen eines Abwassers mittels photosynthetisch aktiver Algen zu schaffen, das ausschließlich zur Bildung von sich absetzenden Algen führt und der Ausbildung von frei schwebenden Algen entgegenwirkt, um so den Aufwand für die Biomasse-Ernte minimieren zu können.

Erfindungsgemäß wird diese Aufgabe durch das Verfahren mit den Merkmalen von Anspruch 1 gelöst. Die Aufgabe wird auch durch die Vorrichtung mit den Merkmalen von Anspruch 7 gelöst. Die Unteransprüche geben jeweils bevorzugte Ausführungsbeispiele an.

Grundgedanke der Erfindung ist es, für die Abwasserreinigung statt frei schwebender Algen ausschließlich photosynthetisch aktive Algen zu verwenden, die im Ruhezustand ein Sediment ausbilden. Insbesondere werden hierfür sich schnell absetzende Algenmischkulturen verwendet.

Die Verwendung von sich schnell absetzenden Algenmischkulturen bedeutet wiederum, dass die bisher verwendeten Röhrensysteme, die - wie beispielsweise in der EP 0 078 033 A1 vorgesehen - zur Kultivierung von frei schwebenden Algen aus vertikal angeordneten Einzelröhren bestehen und weder über eine Verbindung untereinander, noch über eine Umpumpung zwischen den Einzelröhren aufweisen, nicht zur Durchführung des erfindungsgemäßen Verfahrens geeignet sind.

Das erfindungsgemäße Röhrensystem weist daher eine Mehrzahl von horizontal angeordneten Röhren auf, die eine durchgehende Rohrleitung bildend aufsteigend miteinander verbunden sind. Die horizontale Anordnung ermöglicht eine optimale Lichtausbeutung der auf das Röhrensystem scheinenden Sonne, wobei die vertikale Leitung auf ein höheres Niveau die Überleitung in ein Absetzbecken erlaubt, in dem die sich schnell absetzenden Algen gravitätisch geerntet und vom Klarwasser getrennt werden können Darüber hinaus wird durch diese Art der Abwasserbehandlung auch das Aufkommen frei schwebender Algen verhindert.

Insbesondere werden die in dem die horizontal angeordneten, aufsteigend miteinander verbundenen transparenten Röhren von unten nach oben durchgängig kontinuierlich so schnell durchspült, dass sich die bei niedrigerer Geschwindigkeit absetzenden Algenpopulationen nicht absetzen können, sondern in der Wasserphase von unten nach oben gefördert werden. Die Umwälzgeschwindigkeit sollte daher mindestens 0,1 m/s betragen. Für die Länge und Breite des gesamten Röhrensystems bestehen keine prinzipiellen Ober- oder Untergrenzen. Allerdings sollte der Durchmesser der Einzelröhren bevorzugt nicht außerhalb des Bereiches DN 20 mm - DN 200 mm, besonders bevorzugt nicht außerhalb des Bereiches DN 40 mm - 160 mm liegen, in Abhängigkeit von der Dichte der Algenkultur, der Lichtintensität und der Art des zu behandelnden Abwassers.

Der obere Ablauf ist mit einer Sedimentationseinheit integral verbunden, z.B. einem klassischen, aus der Abwassertechnologie bekannten Absetzbecken oder auch einem Parallelplattenabscheider. Aus dem unteren Absetzbereich dieses Absetzbehälters wird das Wasser kontinuierlich mit einer Pumpe entnommen und in die unterste Röhre rezirkuliert. In diese interne Rezirkulation, die sowohl der Ernte als auch dem Verhindern des Absetzens dient, wird das zu behandelnde Abwasser / Grauwasser / Spülwasser / Prozesswasser gefördert, bevorzugt in die unterste Röhre. Genau die gleiche Menge läuft dann aus der Klarwasserablaufrinne des Absetzbehälters als gereinigtes bzw. benutztes Wasser ab und kann abgeleitet oder anderweitig genutzt werden.

Das erfindungsgemäße Verfahren folgt dabei dem Pfropfenstrom-Prinzip: Das zugeführte Abwasser erreicht den Absetzbehälter erst nach Durchlauf der gesamten Röhrenlänge, wodurch erhebliche reaktionskinetische Vorteile erreicht werden.

Erfindungsgemäß ist also ein Verfahren zum Reinigen eines Abwassers mittels photosynthetisch aktiver, im Ruhezustand ein Sediment ausbildender Algen vorgesehen, mit den Schritten a. Zuführen eines Abwassers zu photosynthetisch aktiven, im Ruhezustand ein Sediment ausbildenden Algen, b. Fördern des den Algen zugeführten Abwassers entgegen der Schwerkraft von einem unteren Niveau auf ein höheres Niveau bei gleichzeitiger Lichtexposition des mit den Algen vermengten Abwassers, c. Einleiten des den Algen zugeführten Abwassers vom höheren Niveau in den oberen Bereich eines Absetzbehälters, d. Sedimentierenlassen der Algen im Absetzbehälter und e. Entnehmen des von den Algen durch Sedimentation befreiten Abwassers als gereinigtes Abwasser. Das Entnehmen des gereinigten Abwassers erfolgt insbesondere durch den Überlauf des Absetzbehälters.

Das Verfahren wird bevorzugt kontinuierlich durch Wiederholen der Schritte a. bis e. ausgeführt, wobei wenigstens eine Teilmenge der im Schritt d. sedimentierten Algen bei erneutem Durchlaufen des Verfahrens unter Ausbilden eines Kreislaufs dem Schritt a. zugeführt wird. Andersherum ausgedrückt werden die Algen im Kreislauf geführt, wobei das Abwasser zwischen dem Absetzbehälter und dem Fördervorgang entgegen der Schwerkraft dem Algenkreislauf zugeführt wird.

Insbesondere ist bei der Ausgestaltung als Kreislauf vorgesehen, dass das Volumen des in Schritt a. den Algen zugeführten Abwassers genau dem Volumen des zeitgleich ablaufendem gereinigten Abwassers entspricht.

Vorteilhaft ist es, wenn bei erneutem Durchlaufen des Verfahrens das Zuführen des Abwassers in Schritt a. bei Auftreten einer auf dem höheren Niveau gemessenen vorbestimmten Ammoniumkonzentration unter Aufrechterhalten des reinen Rezirkulations-Kreislaufs ausgesetzt wird bis die Ammoniumkonzentration unterhalb der vorbestimmten Ammoniumkonzentration gesunken ist. So wird sichergestellt, dass die Abwasserreinigung durch Einstellen eines optimalen Verhältnisses von Algen zu Abwasser auch tatsächlich effizient ausgeführt wird.

Um zu vermeiden, dass sich die Algen bereits in den Röhren und nicht nur im Absetzbehälter absetzen, ist vorgesehen, dass die Fördergeschwindigkeit in Schritt b. mindestens 0,1 m/s beträgt. Die Fördergeschwindigkeit ist in jedem Fall so zu bemessen, dass sich die Algen im Röhrensystem nicht absetzen können.

Die Vorrichtung zur Abwasserreinigung zur Durchführung des erfindungsgemäßen Verfahrens sieht entsprechend eine Mehrzahl von horizontal ausgerichteten transparenten Röhren vor, die vertikal übereinander angeordnet und miteinander einen durchgängigen mäanderförmigen Rohrverlauf bildend verbunden sind, und einen Absetzbehälter zur Trennung der photosynthetisch aktiven, im Ruhezustand ein Sediment ausbildenden Algen vom gereinigten Abwasser, mit einem gereinigtes Abwasser abführenden Ablauf und einer die ein Sediment ausbildenden Algen aufnehmenden Trichterspitze vor. Dabei ist die oberste Röhre mit dem oberen Bereich des Absetzbehälters, dem Absetzbehälter mit Algen vermengtes Abwasser zuführend verbunden. Die unterste Röhre hingegen ist über eine die in die Trichterspitze des Absetzbehälters sedimentierenden Algen in die unterste Röhre pumpende Pumpe mit der Trichterspitze des Absetzbehälters verbunden, wobei eine der untersten Röhre Abwasser zuführende Zufuhr vorgesehen ist.

Bevorzugt weist der Absetzbehälter eine aus der Trichterspitze des Absetzbehälters Algenbiomasse aus der Vorrichtung abführende Entnahme auf.

Besonders bevorzugt ist dabei eine Mehrzahl von in verschiedenen Höhen am Absetzbehälter angeordneten Algen-Entnahmestellen vorgesehen, die mit der Pumpe verbunden sind.

Schließlich weist die Vorrichtung nach einem weiteren bevorzugten Ausführungsbeispiel einen im oberen Bereich des Absetzbehälters angeordneten, auf die der untersten Röhre Abwasser zuführende Zufuhr wirkenden Sensor zum Erfassen des Ammoniumgehalts.

Die bevorzugten Aufstellungsorte einer solchen Vorrichtung sind Südwände von Gebäuden. Mit Aufstellung direkt an der Gebäudeaußenwand sind Wind- und ein Kälteschutz gewährleistet. Der Absetzbehälter wird bevorzugt an der Gebäudeinnenwand auf Höhe der am höchsten gelegenen Röhre installiert, um die hydraulischen Verluste zu minimieren.

Um ein Einfrieren des Abwassers bzw. des die Algen enthaltenden wässrigen Mediums bei Minusgraden zu verhindern, kann vor der Röhrenwand beispielsweise eine aus Plexiglas-Doppel-Platten gebildete transparente Fläche aufgestellt werden.

Der mit der Erfindung erreichte Vorteil liegt darin, dass der Aufwand für die Biomasse-Ernte gering ist oder völlig entfällt und darüber hinaus eine zielgerichtete Produktion von im Ruhezustand absetzbaren Algenmischkulturen gewährleistet ist, bei der das Aufkommen von Schwebealgen ausgeschlossen ist.

Die Erfindung wird anhand von in den Zeichnungen dargestellten, besonders bevorzugt ausgestalteten Ausführungsbeispielen näher erläutert. Es zeigen:
- Fig. 1: eine schematische Seitenansicht eines besonders bevorzugt ausgestalteten Ausführungsbeispiels nach der Erfindung; und
- Fig. 2: eine schematische Seitenansicht eines besonders bevorzugt ausgestalteten Absetzbehälters, der im Rahmen der erfindungsgemäßen Vorrichtung bevorzugt Anwendung finden kann.

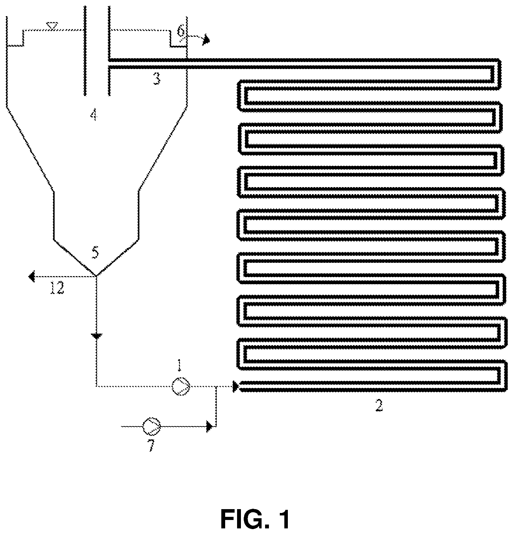

Fig. 1 zeigt eine schematische Seitenansicht eines besonders bevorzugt ausgestalteten Ausführungsbeispiels einer Vorrichtung nach der Erfindung. Die Vorrichtung weist an ihrem unteren Ende eine Pumpe 1 auf, die eine bevorzugt vorgesehene Zirkulation einer wässrigen Kultur von sich leicht absetzenden Algen in der Vorrichtung bewirkt. In diesem Bereich wird der Algenkultur mittels der Zufuhr 7, beispielsweise eine weitere Pumpe, Abwasser zugeführt, sodass Abwasser und Algenkultur miteinander vermengt werden.

Dieses Gemisch aus Abwasser und Algenkultur wird zumindest durch die Pumpe 1 in das aus einer Mehrzahl von horizontal ausgerichteten Röhren, aufsteigende Röhrensystem 2 hineingepumpt, bevorzugt mit einer Strömungsgeschwindigkeit von mindestens mit 0,2 m/s, um das Absetzen der sich schnell absetzenden Algen zu verhindern. Hierbei werden die Algen gemeinsam mit dem Abwasser entgegen der Schwerkraft von einem unteren Niveau auf ein höheres Niveau verbracht, wobei durch die im Röhrensystem 2 ablaufenden auf die Algenmischkultur zurückgehenden biochemischen Prozesse eine Reinigung des Abwassers erfolgt.

Die oberste Röhre führt in den Zulaufbereich 3 des Absetzbehälters 4. Diese ist als Trichterbecken ausgeführt, in dem sich die Algen in der unteren Trichterspitze 5 absetzen und das von Algen befreite und gereinigte Klarwasser aus einem oberen Überlauf 6 abläuft. Die Zufuhr von frischem Abwasser erfolgt über die Zufuhr 7. Daraus ergibt sich, dass bei einer Zufuhr von x m³/Stunde auch genau x m³/Stunde gereinigtes Abwasser aus 6 abläuft. Ohne Abwasserzufuhr läuft die Algenkultur schlicht im Kreis und wird nur zur Biomasseproduktion bzw. Nährstoffaufnahme aus dem im System befindlichen Abwasser genutzt. Mit einer separaten Entnahme 12 kann die produzierte Algenbiomasse entnommen werden. Die hydraulische Aufenthaltszeit im Röhrensystem während der Förderphase sollte im Sommer, also während der Sommermonate, allerdings 48 Stunden nicht überschreiten und möglichst unterhalb von 48 h liegen, weil sich ansonsten Schwebealgen im System etablieren würden.

Eine besondere Ausführungsform der Erfindung betrifft die über eine online Messung der Wasserqualität geregelte Zufuhr von Abwasser. Hierzu wird im Zulaufbereich zum Absetzbehälter 4 kurz vor 3 eine Ammonium-Online-Sonde installiert, die die Abwasserzufuhr wie folgt regelt: Sobald ein frei gewählter Ammoniumwert überschritten wird (z.B. 0,1 mg/l), wird die Abwasserzufuhr unterbrochen. Erst nachdem die nachfolgende Photosynthese zur Unterschreitung dieses Grenzwertes geführt hat, wird die Abwasserzufuhr wieder freigegeben.

Zu den entscheidenden Parametern des Algenwachstums gehört die Algendichte in den Röhren (in g TS pro Liter, TS = Trockensubstanz). Der optimale Dichtewert ist von der Zusammensetzung der Algenmischpopulation und von der Lichtintensität abhängig (und natürlich vom Röhrendurchmesser): Je geringer die Lichtintensität ist, desto geringer ist die optimale Dichte. Dieser optimale Dichtewert liegt zwischen einer zu hohen Dichte, die durch übermäßige Beschattung den Algen-Ertrag mindert und einer zu geringen Dichte, bei der die Lichthemmung den Ertrag reduziert. Empirisch lässt sich für jede Algenmischpopulation die jeweils zugehörige optimale Dichte-Lichtintensitätsfunktion ermitteln. Daraus ergibt sich, dass im Tagesverlauf eine entsprechend geregelte Dichteveränderung zu einer Ertragssteigerung (bzw. verbesserten Abwasserreinigung) führen würde.

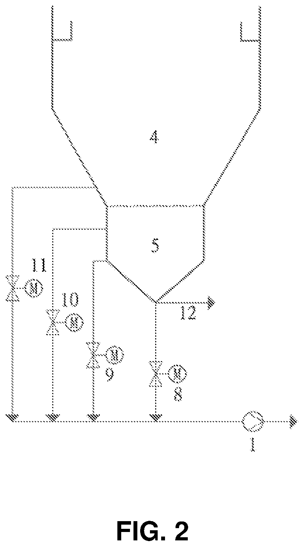

Die Unempfindlichkeit der auf schnelles Absetzen selektierten Algenmischpopulation gegen längere anaerobe Lagerungszeiten sowie der Absetzbehälter mit einer nutzbaren Speichermöglichkeit im unteren Trichterbereich wird genutzt für folgende besondere Ausführungsform der Erfindung:
Die empirisch ermittelte optimale Dichte-Lichtintensitätsfunktion wird in einer Speicherprogrammierbaren Steuerung hinterlegt. Eine TS-Sonde in den Röhren ermittelt online den aktuellen Dichte-Wert der Algenpopulation. Eine Lichtintensitätsmessung ermittelt online die aktuelle Lichtintensität. Die Entnahme der sich absetzenden Algenmischpopulation im Trichterbereich des Absetzbehälters 4 erfolgt, wie Fig. 2 verdeutlicht, geregelt mittels Regelschiebern in unterschiedlichen Höhen am Absetzbehälter 4.

Nach dem höchsten Stand der Sonne zur Mittagszeit fällt die Lichtintensität zum Abend hin ab. Dadurch wird die optimale Dichte immer geringer. Die optimale Einjustierung erfolgt automatisch dadurch, dass nach Abfall der Lichtintensität unter vorgegebenen Grenzwerten, die Entnahme der Algen aus dem Trichterbereich immer weiter oben und damit mit geringerer Algendichte stattfindet. Unter diesen höheren Entnahmestellen setzt sich der Algenschlamm ab und wird zwischengelagert (und gegebenenfalls abgeerntet) bis zum nächsten Anstieg der Lichtintensität, die dann wiederum automatisch zu tieferen Entnahmestellen mit höherer Algendichte führt.

Wie Fig. 2 zeigt, ist der Absetzbehälter 4 bevorzugt mit mehreren Algen-Entnahmestellen 8, 9, 10 und 11 versehen. Bei einer sehr hoher Lichtintensität erfolgt die Rezirkulation in das Röhrensystem mittels der Pumpe 1 über die Entnahmestelle 8 aus dem untersten Trichterbereich. Sobald die Lichtintensität unter den aktuellen und zu dieser Dichte gehörigen Optimalwert fällt, wird automatisch die Entnahmestelle 9 geöffnet und Entnahmestelle 8 geschlossen, entsprechend wird mit den Entnahmestellen 10 und 11 verfahren. Bei einer Entnahme oberhalb der Entnahmestelle 8 reichert sich der Algenschlamm zwischen den Entnahmestellen 8 und 9 an. Dieser hochkonzentrierte Algenschlamm kann dann im Bedarfsfall über die Entnahmestelle 12 abgeerntet werden.

Diese Ernte kann dabei auch wie folgt automatisiert werden: Wenn mit dieser Regelung der höchste Sonnenstand nicht zum Öffnen der Entnahmestelle 8 führt, wird automatisch die Menge zwischen der Entnahmestelle 8 und der dann offenen Entnahmestelle über die Entnahmestelle 12 abgeerntet.

Bevorzugt wird also nur eine Teilmenge der im Schritt d. sedimentierten Algen in Abhängigkeit von der (optischen) Dichte der sedimentierten Algen rezirkuliert.

## Patentansprüche

1. Verfahren zum Reinigen eines Abwassers mittels photosynthetisch aktiver, im Ruhezustand ein Sediment ausbildender Algen, mit den Schritten:
a. Zuführen eines Abwassers zu photosynthetisch aktiven, im Ruhezustand ein Sediment ausbildenden Algen,
b. Fördern des den Algen zugeführten Abwassers entgegen der Schwerkraft von einem unteren Niveau auf ein höheres Niveau bei gleichzeitiger Lichtexposition des mit den Algen vermengten Abwassers,
c. Einleiten des den Algen zugeführten Abwassers vom höheren Niveau in den oberen Bereich eines Absetzbehälters (4),
d. Sedimentierenlassen der Algen im Absetzbehälter (4) und
e. Entnehmen des von den Algen durch Sedimentation befreiten Abwassers als gereinigtes Abwasser.

2. Verfahren nach Anspruch 1, **dadurch gekennzeichnet, dass** das Verfahren kontinuierlich durch Wiederholen der Schritte a. bis e. ausgeführt wird, wobei wenigstens eine Teilmenge der im Schritt d. sedimentierten Algen bei erneutem Durchlaufen des Verfahrens unter Ausbilden eines Kreislaufs dem Schritt a. zugeführt wird.

3. Verfahren nach Anspruch 2, **dadurch gekennzeichnet, dass** das Volumen des in Schritt a. den Algen zugeführten Abwassers dem Volumen des entnommenen gereinigten Abwassers entspricht.

4. Verfahren nach einem der Ansprüche 2 und 3, **dadurch gekennzeichnet, dass** bei erneutem Durchlaufen des Verfahrens das Zuführen des Abwassers in Schritt a. bei Auftreten einer auf dem höheren Niveau gemessenen vorbestimmten Ammoniumkonzentration unter Aufrechterhalten des Kreislaufs ausgesetzt wird bis die Ammoniumkonzentration unterhalb der vorbestimmten Ammoniumkonzentration gesunken ist.

5. Verfahren nach einem der vorhergehenden Ansprüche, **dadurch gekennzeichnet, dass** die Fördergeschwindigkeit in Schritt b. mindestens 0,1 m/s beträgt.

6. Verfahren nach einem der vorhergehenden Ansprüche, **dadurch gekennzeichnet, dass** die hydraulische Aufenthaltszeit der Algen während Schritt b. in den Sommermonaten nicht mehr als 48 h beträgt.

7. Vorrichtung zur Abwasserreinigung zur Durchführung des Verfahrens nach einem der vorhergehenden Ansprüche, mit
- einer Mehrzahl von horizontal ausgerichteten transparenten Röhren (2), die vertikal übereinander angeordnet und miteinander einen durchgängigen mäanderförmigen Rohrverlauf bildend verbunden sind,
- einem Absetzbehälter (4) zur Trennung der photosynthetisch aktiven, im Ruhezustand ein Sediment ausbildenden Algen vom gereinigten Abwasser, mit einem gereinigtes Abwasser abführenden Ablauf und einer die ein Sediment ausbildenden Algen aufnehmenden Trichterspitze (5),
wobei
- die oberste Röhre mit dem oberen Bereich (3) des Absetzbehälters (4), dem Absetzbehälter (4) mit Algen vermengtes Abwasser zuführend verbunden ist,
- die unterste Röhre über eine die in die Trichterspitze (5) des Absetzbehälters (4) sedimentierenden Algen in die unterste Röhre pumpende Pumpe (1) mit der Trichterspitze (5) des Absetzbehälters (4) verbunden ist, und
- eine der untersten Röhre Abwasser zuführende Zufuhr (7) vorgesehen ist.

8. Vorrichtung nach Anspruch 7, **gekennzeichnet durch** eine aus der Trichterspitze (5) des Absetzbehälters (4) Algenbiomasse aus der Vorrichtung abführende Entnahme (12).

9. Vorrichtung nach einem Ansprüche 7 und 8, **gekennzeichnet durch** eine Mehrzahl von in verschiedenen Höhen am Absetzbehälter (4) angeordneten Algen-Entnahmestellen (8, 9, 10, 11), die mit der Pumpe (1) verbunden sind.

10. Vorrichtung nach einem der Ansprüche 7 bis 9, **gekennzeichnet, durch** einen im oberen Bereich des Absetzbehälters (4) angeordneten, auf die der untersten Röhre Abwasser zuführende Zufuhr (7) wirkenden Sensor zum Erfassen des Ammoniumgehalts.

## Claims

1. Method for purifying waste water by means of photosynthetically active algae which form a sediment when in a rest state, comprising the steps:
a. supplying waste water to photosynthetically active algae which form a sediment when in a rest state,
b. conveying the waste water supplied to the algae counter to gravity from a lower level to a higher level while simultaneously exposing the waste water mixed with the algae to light,
c. introducing the waste water supplied to the algae from the higher level into the upper region of a settling container (4),
d. letting the algae sediment in the settling container (4) and
e. removing the waste water, which is released from the algae by sedimentation, as purified wastewater.

2. Method according to claim 1, **characterized in that** the method is carried out continuously by repeating steps a. to e., at least one partial quantity of the algae sedimented in step d. being supplied to step a. when the method is run through again so as to form a circuit.

3. Method according to claim 2, **characterized in that** the volume of the waste water that is supplied to the algae in step a. corresponds to the volume of the purified wastewater that is removed.

4. Method according to either claim 2 or claim 3, **characterized in that** when a predetermined ammonium concentration measured at the higher level occurs when the method is run through again, the supply of the waste water in step a. is suspended while maintaining the circuit until the ammonium concentration has dropped below the predetermined ammonium concentration.

5. Method according to any of the preceding claims, **characterized in that** the conveying speed in step b. is at least 0.1 m/s.

6. Method according to any of the preceding claims, **characterized in that** the hydraulic retention time of the algae during step b. is no more than 48 hours in the summer months.

7. Device for purifying waste water intended for carrying out the method according to any of the preceding claims, comprising
- a plurality of horizontally oriented transparent tubes (2) which are arranged vertically above one another and are connected so as to form a continuous meandering tube course with one another,
- a settling container (4) for separating the photosynthetically active algae, which form a sediment when in a rest state, from the purified waste water, which container has an outlet which conducts away purified water, and a funnel tip (5) which receives the algae that form a sediment,
- the uppermost tube being connected to the upper region (3) of the settling container (4) so as to supply the settling container (4) with waste water mixed with algae,
- the lowermost tube being connected to the funnel tip (5) of the settling container (4) via a pump (1) that pumps the algae which sediments in the funnel tip (5) of the sedimentation container (4) into the lowermost tube, and
- a feed (7) being provided that supplies the lowermost tube with waste water.

8. Device according to claim 7, **characterized by** a removal means (12) which conducts algae biomass away out of the device out of the funnel tip (5) of the settling container (4).

9. Device according to either claim 7 or claim 8, **characterized by** a plurality of algae removal points (8, 9, 10, 11) arranged at different heights on the settling container (4), which points are connected to the pump (1).

10. Device according to any of claims 7 to 9, **characterized by** a sensor for detecting the ammonium content, which sensor is arranged in the upper region of the settling container (4) and acts on the feed (7) which supplies the lowermost tube with waste water.

## Revendications

1. Procédé de purification d'une eau usée au moyen d'algues photosynthétiquement actives formant un sédiment au repos, comprenant les étapes :
a. de fourniture d'une eau usée aux algues photosynthétiquement actives formant un sédiment au repos,
b. de transport de l'eau usée fournie aux algues d'un niveau inférieur à un niveau supérieur en sens opposé à la gravité tout en exposant simultanément l'eau usée mélangée aux algues à la lumière,
c. d'introduction de l'eau usée fournie aux algues se trouvant au niveau supérieur dans la zone supérieure d'un bassin de décantation (4),
d. de sédimentation des algues dans le bassin de décantation (4) et
e. d'extraction de l'eau usée débarrassée des algues par sédimentation, sous la forme d'eau usée purifiée.

2. Procédé selon la revendication 1, **caractérisé en ce que** le procédé est mis en oeuvre en continu par la répétition des étapes a. à e., au moins une quantité partielle des algues sédimentées à l'étape d. étant fournie à l'étape a. lorsque le procédé est à nouveau mis en oeuvre pour former un circuit.

3. Procédé selon la revendication 2, **caractérisé en ce que** le volume de l'eau usée fournie aux algues à l'étape a. est égal au volume de l'eau usée purifiée extraite.

4. Procédé selon l'une des revendications 2 et 3, **caractérisé en ce que**, lors d'une nouvelle mise en oeuvre du procédé, la fourniture de l'eau usée à l'étape a. est suspendue lors de l'apparition d'une concentration d'ammonium prédéfinie mesurée au niveau supérieur en maintenant le circuit jusqu'à ce que la concentration d'ammonium ait diminué en dessous de la concentration prédéfinie.

5. Procédé selon l'une des revendications précédentes, **caractérisé en ce que** la vitesse de transport à l'étape b. est d'au moins 0,1 m/s.

6. Procédé selon l'une des revendications précédentes, **caractérisé en ce que** le temps de séjour hydraulique des algues pendant l'étape b. pendant les mois d'été n'est pas supérieur à 48 heures.

7. Dispositif de purification d'eaux usées pour la mise en oeuvre du procédé selon l'une des revendications précédentes, comportant
- une pluralité de tubes transparents (2) orientés horizontalement, disposés verticalement les uns au-dessus des autres et reliés pour former un trajet de tuyau sinueux continu,
- un bassin de décantation (4) destiné à séparer les algues photosynthétiquement actives formant un sédiment au repos de l'eau usée purifiée, une sortie d'évacuation de l'eau usée purifiée et un bout d'entonnoir (5) recevant les algues formant un sédiment,
- le tuyau le plus élevé étant relié à la zone supérieure (3) du bassin de décantation (4), l'eau usée mélangée à des algues étant acheminée vers le bassin de décantation (4),
- le tuyau le plus bas étant relié au bout d'entonnoir (5) du bassin de décantation (4) par l'intermédiaire d'une pompe (1) pompant les algues sédimentées dans le bout d'entonnoir (5) du bassin de décantation (4) dans le tuyau le plus bas, et
- une alimentation (7) alimentant le tuyau le plus bas en eaux usées étant prévue.

8. Dispositif selon la revendication 7, **caractérisé par** une extraction (12) évacuant la biomasse d'algues du dispositif à partir du bout d'entonnoir (5) du bassin de décantation (4).

9. Dispositif selon les revendications 7 et 8, **caractérisé par** une pluralité de points d'extraction d'algues (8, 9, 10, 11) disposés à différentes hauteurs au niveau du bassin de décantation (4) et reliés à la pompe (1).

10. Dispositif selon l'une des revendications 7 à 9, **caractérisé par** un capteur destiné à détecter la teneur en ammonium, lequel capteur est disposé dans la zone supérieure du bassin de décantation (4) et agit sur l'alimentation (7) alimentant le tuyau le plus bas en eaux usées.
